# EUROPEAN PATENT APPLICATION

(11) **EP 3 326 515 A1**
(43) Date of publication of application: **30.05.2018**
(21) Application number: 16827305.0
(22) Date of filing: 20.07.2016
(51) Int. Cl.: A61B 3/14

(54) **DEVICE AND METHOD FOR RECORDING IMAGES FROM THE OCULAR FUNDUS**

(30) Priority: 21.07.2015 ES 201531070
(71) Applicant: Davalor Salud S.L., 31192 Tajonar (Navarra) (ES); Universitat Politècnica De Catalunya, 08034 Barcelona (ES)
(72) Inventor: PUJOL RAMO, Jaume, 08232 Viladecavalls (Barcelona) (ES); ARASA MARTÍ, José, 08232 Viladecavalls (Barcelona) (ES); VILASECA RICART, Meritxell, 08222 Terrasa (Barcelona) (ES); ARJONA CARBONELL, María Montserrat, 08232 Viladecavalls (Barcelona) (ES)
(74) Representative: Cabinet Plasseraud
(86) International application number: PCT/ES2016/070548
(87) International publication number: WO 2017/013295

(57) **Abstract**

A device and method for recording images from the ocular fundus, comprising:
- means for emitting light, configured to illuminate the ocular fundus by emitting light in the visible, infrared and ultraviolet spectra;
- means for capturing light, configured to capture images from the light reflected by the ocular fundus in various spectral bands within the visible, infrared and ultraviolet spectra;
- a set of optical elements dispose in the optical path established between the means for emitting light and the means for capturing light;
- control means configured to command the means for emitting light, the means for capturing light and the set of optical elements.

## Description

### Technical field

The present invention is related to devices and methods used to determine optical characteristics of the eye, proposing a device that enables recording images of the ocular fundus with the aim of diagnosing pathologies of the retina and optic nerve.

### State of the art

Vision is the gateway of 95% of the information we receive and therefore, the most important route for spatial location, emotional communication and learning (for example, with regard to skills of formal recognition, reading, reading comprehension, etc.), such that it is advisable to periodically explore vision to detect and treat the functional problems and pathologies that may arise.

Among the techniques used in medicine for the exploration of vision, there is retinography, which is based on the analysis of the retina, a layer of tissue sensitive to light that is found inside the eye. To detect the different diseases that can affect the retina, such as diabetic retinopathy, hypertensive retinopathy or pigmentary retinosis, or the optic nerve, different color images are taken of the ocular fundus which are analyzed by a specialist.

Thus, the devices used to analyze the retina are comprised of a light emitter that is responsible for projecting a light beam onto the ocular fundus of the patient, and a camera that is responsible for capturing the light reflected by the ocular fundus. The set of images of the ocular fundus captured by the camera is sent to a viewing device where the specialist analyzes the images to detect possible abnormalities or defects in the vision of the patient.

These devices operate by sending a light beam within the visible spectrum, while the cameras used to capture the images obtain images in various spectral bands of the visible spectrum. However, the information provided by these devices is not sufficient, and in many cases it requires the expertise of the specialist to be able to detect abnormalities of the ocular fundus.

The images of the oculus fundus obtained with these devices have several limitations that make the diagnosis difficult, since the images of the ocular fundus are obtained in a limited (visible) spectral range, with a grayscale band or a maximum of three color bands, mostly in two dimensions, and with time intervals that are very long, such that image details are lost over time. On the other hand, during the capture of the images of the ocular fundus, unwanted reflections are produced which make it difficult to make a final diagnosis of the ocular fundus.

Therefore, it is necessary to have an alternative device to those already used, which enables images of the ocular fundus in various spectral bands, three dimensional images, and video sequences to be obtained, where different characteristics of the retina and the optical nerve can be detected, and it enables images that are not altered by unwanted reflections of the cornea to be obtained.

### Object of the invention

According to the present invention, a device is proposed that enables a hyperspectral analysis of the ocular fundus to be carried out, obtaining continuous recording of images of the ocular fundus taken in various spectral bands within the visible, infrared and ultraviolet spectra, such that the specialist responsible for analyzing the retina of the patient has a large amount of information from the images of the retina to be able to diagnose possible defects or abnormalities thereof.

The device for recording images of the ocular fundus comprises:
- means for emitting light, configured to illuminate the ocular fundus by emitting light in the visible, infrared and ultraviolet spectra;
- means for capturing light, configured to capture images from the light reflected by the ocular fundus in various spectral bands within the visible, infrared and ultraviolet spectra;
- a set of optical elements arranged in the optical path established between the means for emitting light and the means for capturing light; and
- control means configured to command the means for emitting light, the means for capturing light and the set of optical elements.

It is especially relevant to be able to have images of the ocular fundus outside of the visible spectrum, and very especially in spectral bands within the ultraviolet and infrared spectra, since the images of the ocular fundus associated with said spectral bands enable details of the ocular fundus that are not observable in the visible spectrum to be obtained, the specialist therefore having essential information to be able to diagnose possible defects or abnormalities of the retina of the patient.

According to an exemplary embodiment of the invention, the means for emitting light emit monochromatic light, or cuasi-monochromatic light; in other words, they emit in a single wavelength or in a very narrow range of wavelengths. For example, the monochromatic light, or cuasi-monochromatic light, can be emitted by using a set of pixels of a LCD microdisplay or a set of LEDs.

According to another exemplary embodiment of the invention, the means for emitting light emit a broad-spectrum light; in other words, they emit a broad range of wavelengths; in this case, it is provided that the means for capturing light incorporate filters to be able to break down the emitted broad-spectrum light into various spectral bands. In the case of using means for emitting broad spectrum light, the means for capturing light incorporate monochromatic filters, or pseudo-monochromatic filters, such as interference filters, which enable the emitted broad-spectrum light to be separated into various spectral bands. To separate the broad-spectrum light into various spectral bands, tunable wavelength filters can also be used, such as acousto-optic or liquid crystal filters.

It is provided that the means for capturing light are CCD, CMOS InGaAs (indium gallium arsenide) or InSb (indium antimonide) cameras, or they are a matrix of radiation signals by pixels.

The control means are configured to determine the time during which the means for emitting light are emitting a light beam, and the time during which the means for capturing light are capturing the light reflected by the ocular fundus. To do so, a timekeeping device that is operationally connected to the means for emitting and capturing light is used. Thus, the means for emitting light and the means for capturing light are synchronized, and the time elapsed from when the means for emitting light emit a light beam to when the means for capturing light capture said light beam after it has been reflected by the ocular fundus can be determined. Therefore, the layer of the ocular fundus that each reflection comes from can be known, achieving a three-dimensional reconstruction of the ocular fundus.

The set of optical elements of the device for recording the images of the ocular fundus comprise polarizers especially adapted to eliminate the unwanted reflections produced by the cornea, such that said unwanted reflections do not affect the images of the ocular fundus recorded by the means for capturing light. Likewise, the set of optical elements further comprises optical reflectors, such as mirrors or lenses, that direct the light emitted by the means for emitting light. Segmented mirrors are also used which, in addition to directing the light, provide it with a particular structure, such that the alterations produced in the structure of the light once reflected on the ocular fundus can be used by the specialist to determine abnormalities of the ocular fundus. Likewise, the structured light enables the resolution of the images that are obtained from the ocular fundus to be increased.

The device for recording images of the ocular fundus further comprises an aberrometer that is used to measure the ocular aberrations, and an adaptive optical system that subsequently corrects the optical aberrations of the eye of the patient detected by the aberrometer. In this way, the optical aberrations do not affect the images recorded by the means for capturing light.

According to the above, the method for recording images of the ocular fundus used comprises the steps of:
- emitting light in the visible, infrared and ultraviolet spectra onto the ocular fundus;
- capturing the light reflected by the ocular fundus in various spectral bands within the visible, infrared and ultraviolet spectra;
- covering the electromagnetic spectrum from the ultraviolet band to the infrared band; and obtaining a hyperspectral image of the ocular fundus; and
- extracting spectral information from each of the pixels of the hyperspectral image by applying spectrum recovery techniques; and
- obtaining a display in RGB color from the spectral information by applying the sRGB space or techniques such as those based on the analysis of main components or on the compression of spectral regions; and
- repeating the previous steps, covering the electromagnetic spectrum from the ultraviolet band to the infrared band several times; and obtaining a hyperspectral image for each time the electromagnetic spectrum is covered; and
- sequentially arranging the hyperspectral images of the ocular fundus to obtain dynamic recording.

Said dynamic recording enables a video of the ocular fundus to be obtained which enables the specialist to more efficiently diagnose the pathologies of the retina and optic nerve. To obtain a video that provides greater accuracy regarding the behavior of the retina and optic nerve, the time gaps that can appear between two hyperspectral images of the ocular fundus are completed by creating images of the filled ocular fundus obtained by means of image generation algorithms based on the comparison of the results with images obtained from other users.

The light can be intermittently emitted by the means for emitting light in short time intervals, covering the spectrum from infrared to ultraviolet, or it can be continuously emitted, for example, by using white light, which is subsequently broken down by filters into various spectral bands.

The emitted light can illuminate the ocular fundus in its entirety, or it can partially illuminate the ocular fundus. In the case of partially illuminating the ocular fundus by sectors, the means for capturing light obtain a partial image for each section of the illuminated ocular fundus. These partial images of the ocular fundus are overlapped by using image overlapping techniques to obtain a broader image of the ocular fundus, which has more information than the images that capture the ocular fundus in its entirety.

A device for ocular diagnosis is thus obtained, which given its constructive and functional characteristics is preferred for its intended purpose with regards to the determination of the ocular fundus of the patient.

### Description of the figures

Figure 1 shows a block diagram of an exemplary embodiment of the device for recording images of the ocular fundus of the invention.
Figure 2 shows the elements of the device for recording images of the ocular fundus operationally connected to a control unit that governs them.

### Detailed description of the invention

The device for recording images of the ocular fundus comprises means for emitting light that are especially configured to illuminate the ocular fundus of the eye (1) of a patient by emitting light in the visible, infrared and ultraviolet spectra; means for capturing light especially configured to capture images from the light reflected by the ocular fundus in various spectral bands within the visible, infrared and ultraviolet spectra; a set of optical elements arranged in the optical path established between the means for emitting light and the means for capturing light, which can be used to direct, polarize and structure the emitted light; and control means configured to command the means for emitting light, the means for capturing light and the set of optical elements.

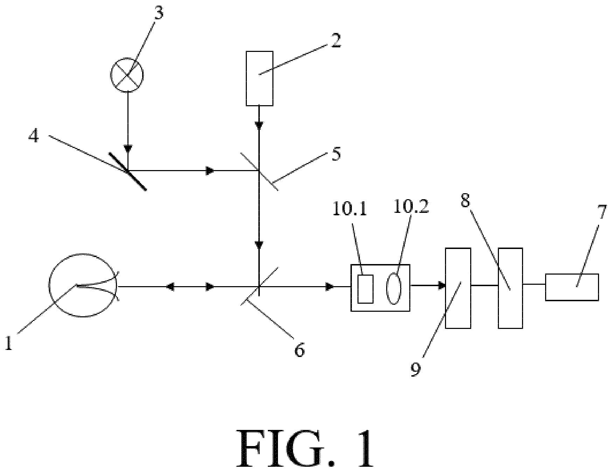

As observed in the exemplary embodiment of Figure 1, the means for emitting light comprise a laser (2) that emits a laser beam to create a point of light that partially illuminates the fundus of the eye (1) and a light source (3) that emits visible, infrared and ultraviolet light.

The light source (3) can intermittently emit visible, infrared and ultraviolet in short time intervals, covering the electromagnetic spectrum from infrared to ultraviolet, or the light source (3) can continuously emit white light, which subsequently breaks down into various spectral bands.

The light emitted by the light source (3) is directed to a micro-actuator (4) that directs the light to a first beam splitter (5), where the light emitted by the light source (3) is associated with the laser beam emitted by the laser (2), a light beam being obtained that is directed to a second beam splitter (6) that sends it in the direction of the eye (1) of the patient to illuminate the ocular fundus of the eye (1).

The second beam splitter (6) also enables the light reflected by the ocular fundus to be directed to a camera (7), which is responsible for recording images from the reflected light of the ocular fundus. The camera (7) can be a CCD, CMOS, InGaAs (indium gallium arsenide) or InSb (indium antimonide) camera or a matrix of radiation signals by pixels. In any case, the camera (7) is responsible for recording images from the reflected light of the ocular fundus in various spectral bands within the visible, infrared and ultraviolet spectra.

According to an exemplary embodiment of the invention, the means for emitting light emit monochromatic light, or cuasi-monochromatic light; in other words, they emit light in a single wavelength or in a very narrow range of wavelengths. According to another exemplary embodiment of the invention, the means for emitting light emit broad spectrum light; in other words, they emit light in a broad range of wavelengths, in which case, before the camera (7) there are filters (8) responsible for separating the light reflected by the ocular fundus into various spectral bands within the visible infrared and ultraviolet spectra. The filters (8) can be made up of monochromatic filters, or pseudo-monochromatic filters, such as interference filters, such that an image of the ocular fundus associated with a spectral band is obtained for each position of the filter. The separation of the light into various spectral bands can also be carried out by putting tunable wavelength filters, such as acousto-optic or liquid crystal filters, in front of the camera (7).

In the exemplary embodiment shown in the figures, the filters (8) that enable the light to be separated into various spectral bans are arranged just in front of the camera (7) once the light has been reflected by the ocular fundus, although they could be arranged in another place in the optical path established between the means for emitting light and the means for capturing light, such as the light source (3) output.

When the light source (3) continuously emits white light, the filters (8) are used to break down said white light into various spectral bands, such that the camera (7) obtains images of the ocular fundus in the various spectral bands that have been broken down by the filters (8).

It is provided that polarizers (9) are used which enable the polarized light coming from the reflection of the cornea to be eliminated, thus obtaining much clearer images of the ocular fundus.

Likewise, it is provided that there is the possibility that the light emitted by the means for emitting light can be oriented by using microactuators, such as segmented micromirrors, or piezoelectric elements, which are arranged along the optical path of the light. The segmented micromirrors, in addition to directing the light to the eye (1), provide the light with a particular structure. The structure of the light is altered after having been reflected on the ocular fundus, such that based on how this alteration occurs, the specialist can detect abnormalities of the retina of the patient. Likewise, the structured light increases the resolution of the images that are obtained from the ocular fundus.

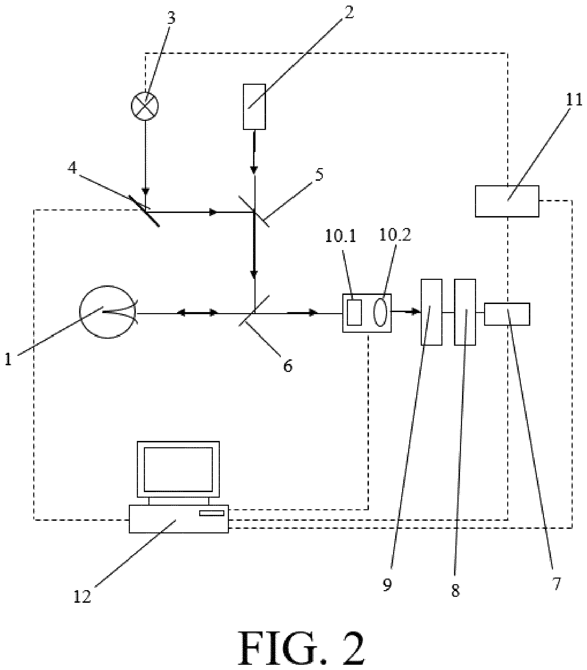

As observed in Figure 2, the device for recording images of the ocular fundus further incorporates an aberrometer (10.1) in front of the camera (7) to measure the optical aberrations of the eye (1), and an adaptive optical system (10.2) based on a matrix of deformable mirrors that enables the aberrations of the eye (1) detected by the aberrometer (10.1) to be corrected. Thus, the images obtained of the ocular fundus are much clearer since they are free of the aberrations of the eye (1).

The means for emitting light, specifically the light source (3), and the means for capturing light, the camera (7), are operationally connected to a timekeeping device (11) that is responsible for determining the time during which the light source (3) emits a light beam to the eye (1) of the patient and the time during which the camera (7) receives the light reflected by the ocular fundus. The means for emitting light and the means for capturing light are synchronized, such that the time elapsed from when the means for emitting light emit a light beam to when the mean for capturing light capture said light beam after it has been reflected by the ocular fundus can be determined. Thus, by recognizing the delay existing between the emission of the light and the reflection thereof by the cornea, the layers of the corner that the reflected light comes from can be known.

The information of the measured times is sent to a control unit, such as a computer (12), where the three-dimensional geometry of the ocular fundus can be reconstructed by using specific image reconstruction algorithms.

The time during which the means for emitting light emit can be controlled by using devices that can be controlled by the computer (12), as an example and not being limitative; and the control of the time of emission can be carried out by MOSFET devices combined with light detectors with an avalanche effect or timers based on quartz oscillators.

Given the above, the method for recording images of the ocular fundus used is thus comprised of the following steps:
- emitting light in the visible, infrared and ultraviolet spectra on the ocular fundus;
- capturing the light reflected by the ocular fundus in various spectral bands within the visible, infrared and ultraviolet spectra;
- covering the bands of the electromagnetic spectrum from the ultraviolet band to the infrared band, and obtaining a hyperspectral image of the ocular fundus;
- extracting spectral information from each of the pixels of the hyperspectral image by applying spectrum recovery techniques;
- obtaining a display in RGB color from the spectral information by applying the sRGB space or techniques such as those based on the analysis of main components or on the compression of spectral regions; and
- repeating the previous steps such that the bands of the electromagnetic spectrum from the ultraviolet band to the infrared band are covered several times, and a hyperspectral image of the ocular fundus, which is an image of the ocular fundus obtained for the spectral bands within the visible, infrared and ultraviolet spectra, is obtained for each time the electromagnetic spectrum mentioned above is covered;
- sequentially arranging the hyperspectral images of the ocular fundus obtained in the successive times the electromagnetic spectrum is covered to obtain dynamic recording in the form of a video.

The means for emitting light can intermittently emit light in short time intervals, sequentially covering the electromagnetic spectrum from infrared to ultraviolet, such that an image of the ocular fundus is obtained for each spectral band within the visible, infrared and ultraviolet spectra.

The means for emitting light can also continuously emit white light, and the light can be broken down by filters (8) into various spectral bands, and an image of the ocular fundus is obtained for each spectral band.

The light emitted on the eye (1) can completely illuminate the ocular fundus, or it can partially illuminate the ocular fundus; in other words, the ocular fundus is illuminated by sectors, such that by applying automatic recognition techniques of image overlapping, a broader image of the ocular fundus of the eye (1) of the patient can be obtained.

Thus, a sequence of images is obtained, each of which corresponds to a specific wavelength within the electromagnetic spectrum that ranges from infrared to ultraviolet. Thus, all the bands of the spectrum are covered from infrared to ultraviolet, where the specialist has a large amount of information to be able to detect abnormalities of the retina, and by combining the successive hyperspectral images obtained, dynamic video recording of the fundus of the eye (1) can be obtained.

To obtain a video that provides greater accuracy regarding the behavior of the retina and optic nerve, the time gaps that can be created between two hyperspectral images of the ocular fundus are completed by creating hyperspectral images of the filled ocular fundus obtained by means of image generation algorithms that are based on the images obtained in previous analyses of other users.

## Claims

1. A device for recording images of the ocular fundus, **characterized in that** it comprises:
- means for emitting light, configured to illuminate the ocular fundus by emitting light in the visible, infrared and ultraviolet spectra;
- means for capturing light, configured to capture images from the light reflected by the ocular fundus in various spectral bands within the visible, infrared and ultraviolet spectra;
- a set of optical elements arranged in the optical path established between the means for emitting light and the means for capturing light; and
- control means configured to command the means for emitting light, the means for capturing light and the set of optical elements.

2. The device for recording images of the ocular fundus according to claim 1, **characterized in that** the means for emitting light emit monochromatic light, or quasi-monochromatic light.

3. The device for recording images of the ocular fundus according to the previous claim, **characterized in that** the means for emitting light that emit monochromatic light, or quasi-monochromatic light, comprise a set of pixels of a LCD microdisplay or a set of LEDs.

4. The device for recording images of the ocular fundus according to claim 1, **characterized in that** the means for emitting light emit broad spectrum light.

5. The device for recording images of the ocular fundus according to claims 1 and 4, **characterized in that** the means for capturing light incorporate filters (8) to separate the light into various spectral bands.

6. The device for recording images of the ocular fundus according to the preceding claim, **characterized in that** the filters (8) are monochromatic filters, or pseudo-monochromatic filters, such as interference filters.

7. The device for recording images of the ocular fundus according to claim 5, **characterized in that** the filters (8) are tunable wavelength filters, such as acousto-optic or liquid crystal filters

8. The device for recording images of the ocular fundus according to claim 1, **characterized in that** the means for capturing light are CCD, CMOS, InGaAs or InSb cameras or a matrix of radiation signals by pixels.

9. The device for recording images of the ocular fundus according to claim 1, **characterized in that** the control means are configured to determine the time elapsed from when the means for emitting light emit a light beam to when the means for capturing light capture said light beam after it has been reflected by the ocular fundus can be determined.

10. The device for recording images of the ocular fundus according to claim 1, **characterized in that** the set of optical elements comprise polarizers adapted to polarize the light reflected by the ocular fundus.

11. The device for recording images of the ocular fundus according to claim 1, **characterized in that** the set of optical elements comprise segmented mirrors that provide the emitted light with a structure.

12. The device for recording images of the ocular fundus according to claim 1, **characterized in that** the set of optical elements comprise optical reflectors that orient the light from when it is emitted by the means for emitting light until when it is reflected on the ocular fundus and is captured by the means for capturing light.

13. The device for recording images of the ocular fundus according to claim 1, **characterized in that** the device incorporates an aberrometer (10.1) and an adaptive optical system (10.2) used to measure and correct optical aberrations of the eye (1) of the patient.

14. A method for recording images of the ocular fundus by using the device of the preceding claims, **characterized in that** it comprises the steps of:
- emitting light in the visible, infrared and ultraviolet spectra onto the ocular fundus;
- capturing the light reflected by the ocular fundus in various spectral bands within the visible, infrared and ultraviolet spectra;
- covering the electromagnetic spectrum from the ultraviolet band to the infrared band; and obtaining a hyperspectral image of the ocular fundus;
- extracting spectral information of each of the pixels of the hyperspectral image by applying spectrum recovery techniques;
- obtaining a display in RGB color from the spectral information by applying the sRGB space or techniques such as those based on the analysis of main components or on the compression of spectral regions;
- repeating the previous steps, covering the electromagnetic spectrum from the ultraviolet band to the infrared band several times; and obtaining a hyperspectral image for each time the electromagnetic spectrum is covered; and
- sequentially arranging the hyperspectral images of the ocular fundus to obtain dynamic recording.

15. The method for recording images of the ocular fundus according to claim 14, **characterized in that** the light is intermittently emitted onto the ocular fundus, covering the electromagnetic spectrum from infrared to ultraviolet, and an image of the ocular fundus is obtained for each spectral band within the visible, infrared and ultraviolet spectra.

16. The method for recording images of the ocular fundus according to claim 14, **characterized in that** white light is continuously emitted onto the ocular fundus, and the light is broken down by filters into various spectral bands, and an image of the ocular fundus is obtained for each spectral band.

17. The method for recording images of the ocular fundus according to claim 14, **characterized in that** the emitted light illuminates the ocular fundus in its entirety or partially.
